# EUROPÄISCHE PATENTSCHRIFT

(11) **EP 0 701 587 B1**
(45) Veröffentlichungstag und Bekanntmachung des Hinweises auf die Patenterteilung: **06.08.1997**
(21) Anmeldenummer: 93909539.4
(22) Anmeldetag: 03.05.1993
(51) Int. Cl.: C08L 1/28, C08L 3/00, C08L 5/00, A61L 15/28, A61L 15/60

(54) **POLYMERZUSAMMENSETZUNGEN, HERSTELLUNG VON POLYMERZUSAMMENSETZUNGEN, INSBESONDERE ABSORPTIONSMATERIALIEN UND DEREN VERWENDUNG**
POLYMER COMPOSITIONS AND THEIR PRODUCTION, IN PARTICULAR ABSORBENT MATERIALS, AND THEIR USE
COMPOSITIONS POLYMERES, PRODUCTION DE COMPOSITIONS POLYMERES, NOTAMMENT DE MATERIAUX ABSORBANTS, ET LEUR UTILISATION

(43) Veröffentlichungstag der Anmeldung: 20.03.1996
(73) Patentinhaber: Stockhausen GmbH & Co. KG, 47805 Krefeld (DE)
(72) Erfinder: GÜNTHER, Uwe, 75392 Deckenpfronn (DE); KLIMMEK, Helmut, D-4150 Krefeld 1 (DE); BRÜGGEMANN, Helmut, 47829 Krefeld (DE)
(74) Vertreter: Klöpsch, Gerald, Dr.-Ing. Patentanwalt
(86) Internationale Anmeldenummer: EP9301062
(87) Internationale Veröffentlichungsnummer: WO9425521

(56) Entgegenhaltungen:
- EP-A- 0 405 993
- EP-A- 0 481 225
- EP-A- 0 481 226
- DE-A- 4 206 850
- US-A- 5 137 563

## Beschreibung

Die Erfindung betrifft Polymermaterialzusammensetzungen sowie die Herstellung einer Polymerzusammensetzung und insbesondere Absorptionsmaterialien, die überwiegend auf nachwachsenden Rohstoffen basiert und somit grundsätzlich auch biologisch abbaubar sind. Aufgrund der überwiegend nativen Herkunft enthalten die Absorber keine oder deutlich geringere Mengen an Restmonomeren als Absorber auf Polyacrylatbasis. Die erfindungsgemäßen Absorber besitzen eine vergleichsweise hohe Aufnahmekapazität und -geschwindigkeit, auch unter Druck, für Wasser und wäßrige Lösungen, zeigen keine Neigung zum Gelblocking (Gelblocking: Beim Kontakt mit Wasser verkleben die äußeren Schichten des Absorbers und verhindern ein weiteres Vordringen der Flüssigkeit in den Absorber) und sind mechanisch stabil (bezüglich der Entmischung in die Einzelkomponenten). In gequollenem Zustand separieren sie in einzelne Partikel, sie sind nicht wäßrig und weisen eine sehr hohe Gelstabilität auf. Die Erfindung betrifft ferner ein Verfahren zu ihrer Herstellung und ihre Verwendung als Faser, Film, Pulver oder Granulat zur Absorption von Wasser, wäßrigen Lösungen oder wäßrigen Dispersionen und Körperflüssigkeiten, in Hygienemitteln wie Tampons oder Windeln und Tierhygieneartikeln, in chemische-technischen Produkten wie beispielsweise Verpackungsmaterialien, insbesondere für Fleisch und Fisch, in Kulturgefäßen sowie zur Bodenverbesserung und als Kabelummantelungen.

Die meisten der heute verwendeten Absorptionsmaterialien, auch Superabsorber genannt, die in der Lage sind, in kurzer Zeit große Flüssigkeitenmengen (Wasser, Urin) aufzunehmen, stellen in erster Linie schwach vernetzte Polyacrylate dar und basieren somit nicht auf nachwachsenden Rohstoffen und sind vergleichsweise unzureichend bzw. überhaupt nicht biologisch abbaubar.

Im Bestreben, Superabsorber aus nachwachsenden Rohstoffen aufzubauen, wurde wie in der DE-PS 2612846 beschrieben, Acrylsäure auf Polysaccharide, wie z.B. auf Maisstärke, aufgepfropft. Dabei können allerdings nur geringe Mengen an Polysacchariden (bis max. 25 %) eingesetzt werden, da ansonsten drastische Verschlechterungen der Absorptionseigenschaften erhalten werden.

Genauso können durch die Einarbeitung von Polysacchariden ins Polymerisationsgel von Polyacrylaten, wie in den DE-OS'en 40 29 591, 40 29 592 und 40 29 593 beschrieben wird, die Polyacrylate nur bis zu max. 25 % ersetzt werden, ohne eine deutliche Verschlechterung des Aufnahmevermögens und anderer Eigenschaften der resultierenden Superabsorber zu erhalten, auch wenn zusätzlich diverse Hilfsstoffe wie Fasern und z.B. Aluminiumvernetzer zugesetzt werden. Die Polysaccharide werden als Bausteine für die Absorber gesehen, um biologisch abbaubare Einheiten zu erhalten.

Die DE-PS 3132976 beschreibt die Vermischung von Polyacrylsäure mit Polysacchariden in Pulverform und in Lösung, wobei die Hülle der Absorberteilchen der Gemische mit Aluminiumvernetzern wie Al(OH)₂OOCCH₃ * 1/3 H₃BO₃ vernetzt wird. Demnach sind auch mit diesem Verfahren keine Superabsorber darstellbar, die zu über 60 % aus nachwachsenden Rohstoffen bestehen.

In den bislang nach dem heutigen Stand der Technik beschriebenen Verfahren besitzen die Polysaccharide keinen entscheidenen Beitrag als Absorptionskomponente.

Zahlreiche Veröffentlichungen wie die DE-OS 2634539 beschreiben die Herstellung von Carboxymethylcellulose-Absorbern, also von Materialien, die prinzipiell biologisch abbaubar sind, durch eine Vernetzung der Carboxymethylcellulose mit diversen Vernetzern im wäßrigen System. Diese Absorber zeigen allerdings ein starkes Gelblocking.

In der US-A 4 959 341 wird die Herstellung eines auf Carboxymethylcellulose basierenden Absorbers beschrieben, der aus einer Mischung von Carboxymethylcellulose, Cellulosefasern, einer hydrophoben Komponente und Al(OH)₂OOCCH₃ * 1/3 H₃BO₃ als Vernetzer besteht, wobei der Aluminiumvernetzer eine Vernetzung der Carboxymethylcellulose während der Flüssigkeitsaufnahme bewirkt.

Diese Absorber besitzen gute Absorptionseigenschaften, zeigen aber Blockphänomene. Zudem werden diese Absorber durch mechanische Belastungen, wie Absiebung oder Förderung, leicht entmischt, so daß sie nicht mehr als homogenes Produkt vorliegen, was ihre Einsatzmöglichkeiten stark einschränkt.

In der EP-PS 0 201 895 wird ebenfalls die Herstellung eines Absorbers, der auf Carboxymethylcellulose basiert, beschrieben. Allerdings wird bei der Herstellung dieser Absorber in einer wäßrigen Lösung gearbeitet, in der die Carboxymethylcellulose nur in geringer Konzentration vorliegt. Weiterhin werden bei der Herstellung größere Mengen an organischen Lösungsmitteln wie Aceton, Methanol usw. benötigt. Die Herstellung dieser Carboxymethylcellulose-Absorber erfordert zudem einen hohen Zeitaufwand. Die Absorber selbst zeigen Blockphänomene und eine niedrige Gelstärke.

Während bei der Entwicklung der Superabsorber zunächst allein das sehr hohe Quellvermögen bei Kontakt mit Flüssigkeit, auch freie Quellkapazität genannt, im Vordergrund stand, hat sich später gezeigt, daß es nicht nur auf die Menge der absorbierten Flüssigkeit ankommt, sondern auch auf die Gelfestigkeit. Absorptionsvermögen oder auch Quellvermögen oder freie Quellkapazität genannt einerseits und Gelfestigkeit bei einem vernetzten Polymer andererseits stellten jedoch gegenläufige Eigenschaften dar, wie bereits aus dem US-A 3,247,171 (DOW/WALKER) bekannt ist, ferner aus der US-PS Re 32, 649. Das bedeutet, daß Polymere mit besonders hohem Absorptionsvermögen nur eine geringe Festigkeit des gequollenen Gels aufweisen mit der Folge, daß das Gel unter einem angewendeten Druck (z. B. Körperdruck) deformierbar ist und die weitere Flüssigkeitsverteilung und -aufnahme verhindert. Nach der US-PS Re 32,649 soll daher ein ausgewogenes Verhältnis zwischen Absorptionsvermögen (Gelvolumen) und Gelstärke angestrebt werden, damit bei der Verwendung derartiger Superabsorber in einer Windelkonstruktion Flüssigkeitsaufnahme, Flüssigkeitstransport, Trockenheit der Windel und der Haut gewährleistet sind. Dabei kommt es nicht nur darauf an, daß das Polymer Flüssigkeit unter nachfolgender Einwirkung eines Drucks zurückhalten kann, nachdem das Polymer frei quellen konnte, sondern auch darauf, Flüssigkeiten auch gegen einen gleichzeitigen, d.h. während der Flüssigkeitsabsorption, ausgeübten Druck aufzunehmen, wie es unter praktischen Gegebenheiten geschieht, wenn ein Baby oder eine Person auf einem Sanitärartikel sitzt oder liegt oder wenn es, z.B. durch Beinbewegung, zur Entwicklung von Scherkräften kommt. Die spezifische Absorptionseigenschaft wird in der EP-A-0 339 461 auf Seite 5 bis 7 als Aufnahme unter Druck ("Absorption Under Load" = "AUL") bezeichnet.

Der vorliegenden Erfindung lag die Aufgabe zugrunde, eine Polymerzusammensetzung, insbesondere einen Absorber, bereitzustellen und herzustellen, der die zuvor beschriebenen Mängel nicht aufweist und der die folgende Eigenschaften besitzt:
a) Der Absorber soll überwiegend aus Komponenten nativen Ursprungs bestehen und damit grundsätzlich auch biologisch abbaubar sein.
b) Die Absorber sollen eine hohe mechanische Festigkeit aufweisen, sie dürfen sich beim Sieben oder z.B. bei einer Schneckenförderung nicht in ihre einzelnen Komponenten auftrennen.
c) Die Absorber sollen eine vergleichsweise hohe Aufnahmegeschwindigkeit und -kapazität für Wasser und wässrige Lösungen besitzen.
d) Der Gehalt an Restmonomeren soll deutlich geringer liegen als bei herkömmlichen Absorbern auf Basis von Polyacrylaten.
e) Die Absorber sollen im gequollenen Zustand eine sehr hohe Gelstabilität aufweisen; dabei sollen die Absorberkörner separiert, in einzelnen Partikeln vorliegen.
f) Sie dürfen nicht zum Gelblocking neigen.
g) Die Absorber sollen eine hohe Aufnahmegeschwindigkeit und Aufnahmekapazität unter Druck für Wasser und wäßrige Lösungen besitzen.

Weitere Aufgabe ist die Bereitstellung einer wirkstoffenthaltenden Zusammensetzung, deren Herstellung und Verwendung.

Erfindungsgemäß erfolgt die Lösung der ersten Aufgabe durch eine Polymerzusammensetzung und ein Herstellungsverfahren für eine Polymerzusammensetzung, insbesondere ein Absorptionsmittel, die (das) im wesentlichen aus vier Komponenten besteht:
- einer Komponente A, die auf nachwachsenden speziellen Rohstoffen basiert,
- einer Komponente B, die aus einem speziellen wasserquellbaren Polymeren besteht,
- einem Matrixmaterial,
- einem ionischen oder kovalenten Vernetzer,
- und gegebenenfalls eines Antiblockingmittels.

Die vorliegende Erfindung betrifft eine Polymerzusammensetzung, insbesondere Absorbtionsmaterialzusammensetzung, im wesentlichen bestehend aus
70 bis 99,99 Gew.-% eines wasserlöslichen und/oder wasserquellbaren Polysaccharids oder Polysaccharidderivats, das gegebenenfalls durch Vernetzung modifiziert worden ist (Komponente A) und
0,01 bis 30 Gew.-% eines wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(anhydrid), Itaconsäure(anhydrid), Fumarsäure, Vinylsulfonsäure und/oder 2-Acrylamido-2-methylpropansulfonsäure, den Amiden, den N-Alkylderivaten, den N,N'-Dialkylderivaten, den hydroxylgruppenhaltigen Estern und aminogruppenhaltigen Estern dieser polymerisationsfähigen Säuren, wobei 0 bis 98 Gew.-% der Säuregruppen dieser Säuren neutralisiert sind und wobei diese Polymere und /oder Copolymere durch eine mindestens bifunktionelle Verbindung vernetzt sind (Komponente B) sowie
0,1 bis 30 Gew.-%, bezogen auf die polymeren Komponenten A und B eines organischen Matrixmaterials mit einem Schmelz- bzw. Erweichungspunkt unterhalb von 180° C zur Verhinderung der Entmischung und des Gelblocking
0,001 bis 10 Gew.-%, bezogen auf die beiden polymeren Komponenten A und B, eines ionischen und/oder kovalenten Vernetzers
   und
0 bis 50 Gew.-%, bezogen auf die polymeren Komponenten A + B, wenigstens eines Antiblockingmittels auf Basis natürlicher und/oder synthetischer Fasern und/oder Materialien mit großer Oberfläche, erhältlich durch Zusammenbringen der Komponente B mit der Komponenten A in wäßrigem Medium, anschließende Trocknung und Mahlung, Hinzumischung der weiteren Komponenten, Mischung bis zur Homogenität, Durchführung einer Wärmebehandlung und gegebenenfalls nach Zugabe des Vernetzers zwecks dessen Fixierung durch die Matrix eine abschließende Wärmebehandlung.

Die vorliegende Erfindung betrifft somit weiterhin ein Verfahren zur Herstellung einer Polymerzusammensetzung, insbesondere eines Absorbermaterials, im wesentlichen bestehend aus 70 - 99,99 Gew.-% einer Komponente A, bestehend aus wasserlöslichen und/oder wasserquellbaren Polysacchariden oder deren Derivaten, die gegebenenfalls durch Vernetzung modifiziert worden sind und 0,01 - 30 Gew.-% einer Komponente B, bestehend aus wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(-anhydrid), Itakonsäure(-anhydrid), Fumarsäure, Vinylsolfonsäure und/oder 2-Acrylamido-2-methylpropansulfonsäure sowie den Amiden, N-Alkylderivaten, den N,N'-Dialkylderivaten, hydroxylgruppenhaltigen Estern und aminogruppenhaltigen Estern dieser polymerisationsfähigen Säuren, wobei 0 - 98 % der Säuregruppen dieser Säuren neutralisiert sind, und wobei diese Polymere und/oder Copolymere durch eine mindestens bifunktionelle Verbindung vernetzt sind, als polymere Komponenten, sowie 0,1 - 30 Gew.-%, bezogen auf diese polymeren Komponenten, eines organischen Matrixmaterials mit einem Schmelzpunkt bzw. Erweichungspunkt unterhalb von 180°C zur Verhinderung der Entmischung und des Gelblocking, 0,001 - 10 Gew.-%, bezogen auf diese polymeren Komponenten, eines ionischen oder kovalenten Vernetzers, und ggf. 0 - 50 Gew.-%, bezogen auf diese polymeren Komponenten, wenigstens eines Antiblockingmittels auf Basis natürlicher und/oder synthetischer Fasern und/oder Materialien mit großer Oberfläche, dadurch gekennzeichnet, daß man diese(s) erhält, indem man die Komponente B mit der Komponente A in wäßrigem Medium zusammenbringt und anschließend trocknet und mahlt, die weiteren Komponenten hinzugibt, bis zur Homogenität mischt und eine Wärmebehandlung durchführt und gegebenenfalls bei Zugabe des Vernetzers nach dieser Wärmebehandlung diesen mit der Matrix durch eine abschließende Wärmebehandlung fixiert.

Dabei wurde überraschenderweise gefunden, daß bei der Herstellung ein geringfügiger Zusatz von Komponente B zur Komponente A eine deutliche Verbesserung der Absorptionseigenschaften bewirkt. Da nur geringe Zusätze an Komponente B notwendig sind, liegt damit der Restmonomerengehalt an z. B. Acrylsäure eines solchen Absorbers deutlich geringer als bei Absorbern auf Polyacrylatbasis.
Die Einarbeitung der Komponente B in die Komponente A wird beispielsweise durch gemeinsames Aufquellen dieser in Wasser oder einer wäßrigen Lösung und anschließendes Trocknen erreicht. Überraschenderweise führt dieses Verfahren zu einer deutlichen Erhöhung des AUL - Wertes; außerdem kann überraschenderweise der Anteil der Komponente B deutlich reduziert werden, ohne daß die Absorptionseigenschaften des Produktes beeinträchtigt werden.
Ferner wurde überraschenderweise gefunden, daß durch den Zusatz eines Feststoffes, der als Matrix für das Absorbersystem fungiert, in Kombination mit dem Polymerabsorptionsmittel, einer Mischung aus den Komponenten A und B, und einem ionischen Vernetzer, ein Absorptionsmittel herstellbar ist, das eine hohe Aufnahmegeschwindigkeit und -kapazität für Wasser und wässrige Lösungen sowie eine verbesserte mechanische Festigkeit hinsichtlich der Entmischung der einzelnen trockenen Partikel besitzt. Weiterhin liegen die Gele dieses Absorbersystems separiert in einzelnen Partikeln vor.

Außerdem zeigen diese Absorber überraschenderweise, neben den oben genannten Eigenschaften, eine Gelstärke, die deutlich höher liegt, als die von Absorbern, die auf Polyacrylsäurebasis aufbauen.

Als Komponente A sind wasserlösliche und wasserquellbare Polymere auf der Basis von Polysacchariden und deren Derivate geeignet, wie Guar, Carboxymethylguar, Xanthan, Alginate, Gummi Arabicum, Hydroxyethyl- oder Hydroxypropylcellulose, Carboxymethylcellulose und andere Cellulosederivate, Stärke und Stärkederivate wie Carboxymethylstärke und Mischungen aus den einzelnen Polysacchariden. Die bevorzugten Polymeren sind Guar sowie die anionischen Derivate von Stärke, Guar und Cellulose, wobei Carboxymethylcellulose ein besonders bevorzugtes Material darstellt.

Die aufgeführten Polymeren der Komponente A können durch eine Vernetzung modifiziert werden, um ihre Wasserlöslichkeit zu reduzieren und bessere Quelleigenschaften zu erreichen. Die Vernetzung kann sowohl im gesamten Polymeren stattfinden oder aber auch nur an der Oberfläche der einzelnen Polymerpartikel.

Die Umsetzung der Polymeren kann mit ionischen Vernetzern wie z.B. Calcium-, Aluminium-, Zirkon-, Eisen (III)- und Titanverbindungen erfolgen. Ebenso ist die Umsetzung mit polyfunktionellen Carbonsäuren wie Citronensäure, Schleimsäure, Weinsäure, Äpfelsäure, Malonsäure, Bernsteinsäure, Glutarsäure, Adipinsäure, mit Alkoholen wie Polyethylenglykole, Glycerin, Pentaerythrit, Propandiole, Saccharose, mit Kohlensäureestern wie Ethylen- und Propylencarbonat, mit Aminen wie Polyoxypropylenamine, mit Epoxiverbindungen wie Ethylenglykoldiglydcidylether, Glykoldi- oder triglycidylether und Epichlorhydrin, mit Säureanhydriden wie Bernsteinsäureanhydrid und Maleinsäureanhydrid, mit Aldehyde und mehrfunktionelle (aktivierte) Olefine wie Bis-(acrylamido)-essigsäure und Methylenbisacrylamid möglich. Ebenso kommen natürlich Derivate der genannten Verbindungsklassen in Betracht sowie heterofunktionelle Verbindungen mit verschiedenen funktionellen Gruppen der oben genannten Verbindungsklassen.

Als Komponente B sind wasserquellbare synthetische Polymere oder Copolymere in erster Linie auf der Basis von (Meth) Acrylsäure und weiterhin auf der Basis von (Meth) Acrylnitril, (Meth) Acrylamid, Vinylacetat, Vinylpyrrolidon, Vinylpyridin, Maleinsäure, Maleinsäureanhydrid, Itakonsäure, Itakonsäureanhydrid, Fumarsäure, Vinylsulfonsäure, 2-Acrylamido-2-methylpropansulfonsäure, sowie die Amide, ihre N und N,N'-Dialkylderivate, hydroxylgruppenhaltige Ester und aminogruppenhaltige Ester der polymerisationsfähigen Säuren geeignet. Bevorzugt sind vernetzte, teilweise neutralisierte Polyacrylate.

Es können bis zu 98 %, vorzugsweise 50 - 80 % der Säuregruppen neutralisiert sein.

Die Polymeren können durch einen mindestens bifunktionellen Vernetzer vernetzt sein.

Die Herstellung dieser vorstehenden Polymeren erfolgt nach bekannten Verfahren (DE-PS 27 06 135, DE-OS 40 15 085). Ein besonders bevorzugtes Material als Komponente B stellen Polyacrylate dar, z.B. die von der Chemischen Fabrik Stockhausen GmbH hergestellten FAVOR^{R}-Typen.

Die Komponenten A und B können chemisch, d.h. über Esterbindungen bzw. durch einen der angeführten Vernetzer oder physikalisch, d.h. im Sinne eines "Interpenetrating Networks" (IPN) miteinander verknüpft sein.

Als Matrix sind organische Feststoffe geeignet, die unter 180°C schmelzen bzw. erweichen und vorzugsweise bei Raumtemperatur eine weiche Konsistenz besitzen, wie z.B. Triglycerinmonostearat. Ebenso sind hochviskose Flüssigkeiten wie z.B. Rizinusöl geeignet.

Vorzugsweise sind als Matrix Polycaprolactone geeignet, wie TONE 0230 und 0240 von Union Carbide, die auch modifiziert sein können, wie z.B. durch eine Umsetzung mit Maleinsäureanhydrid.

Durch die Matrix erhält das Absorbersystem vermutlich durch chemische und/oder physikalische Wechselwirkungen eine höhere mechanische Festigkeit, wodurch die Entmischung der Einzelkomponenten durch Transportvorgänge, wie z.B. mittels einer Förderschnecke, oder durch Absiebvorgänge stark vermindert wird. Dadurch kann ein Absorptionsmittel hergestellt werden, das nicht nur hohe Absorptionswerte besitzt, sondern nach einer Konfektionierung bzw. nach Einarbeitung in seinem Verwendungsbereich als homogeneres und somit wirksameres System vorliegt.
Außerdem bewirkt das Einbetten des Absorptionsmaterials in der Matrix überraschenderweise eine deutliche Reduzierung bzw. eine gänzliche Beseitigung des Gelblocking, somit ist eine hohe Aufnahmegeschwindigkeit im ganzen Absorber gewährleistet. Weiterhin wird durch die Matrix der Vernetzer fest an der Oberfläche der einzelnen Absorberpartikel fixiert. Die Granulierung von Superabsorberfeinstäuben durch Agglomerierungshilfsmittel wird in den Beispielen der
DE-PS 37 41 157 und DE-PS 39 17 646 beschrieben. Die so hergestellten Produkte besitzen eine hohe Aufnahmegeschwindigkeit für Wasser und wäßrige Lösungen. Diese Produkte bestehen jedoch nur aus Polyacrylaten und sind folglich vergleichsweise unzureichend bzw. überhaupt nicht biologisch abbaubar. Die Agglomeriesationsmittel dienen nur zur Granulierung eines Produktes und keinesfalls als Matrixmaterial.

Die Anti-Blocking-Mittel vermindern ebenfalls Gelblocking; sie bewirken also eine schnellere und bessere Flüssigkeitsaufnahme und sorgen dafür, daß die Gele separiert, d.h. in einzelnen Partikeln vorliegen.
Geeignete Anti-Blocking-Mittel sind bekanntermaßen (vgl. DE-PS 31 41 098 und DE-PS 33 13 344) Fasermaterialien und andere Materialien mit großer Oberfläche.

Die Fasern können natürliche oder synthetische Fasern sein, wie z.B. Woll-, Baumwoll-, Seiden- und Cellulosefasern, bzw. Polyamid-, Polyester-, Polyacrylnitril-, Polyurethanfasern, Fasern von Olefinen und deren Substitutionsprodukten sowie Polyvinylalkoholfasern und deren Derivate. Beipiele für anorganische Materialien sind Bentonite, Zeolithe, Aerosile und Aktivkohlen.

Geeignete Vernetzer sind Verbindungen, welche die oben beschriebenen Polymeren in einen Zustand überführen, in dem die Wasserlöslichkeit reduziert wird, das Saugvermögen verbessert und die Blockphänomene vermindert werden.

Als ionische Vernetzer sind Metallverbindungen geeignet, die mit den funktionellen Gruppen der Polymere in Wechselwirkung treten können. Besonders bevorzugt sind Magnesium-, Calcium-, Aluminium-, Zirkon-, Eisen-, Titan- und Zinkverbindungen, die eine sehr gute Wasserlöslichkeit besitzen, wie die Salze von Carbonsäuren und anorganischen Säuren.
Bevorzugte Carbonsäuren sind Essigsäure, Milchsäure, Salicylsäure, Propionsäure, Benzoesäure, Fettsäuren, Malonsäure, Bernsteinsäure, Glutarsäure, Adipinsäure, Citronensäure, Weinsäure, Äpfelsäure und Schleimsäure. Bevorzugte anorganische Anionen sind Chloride, Bromide, Hydrogensulfate, Sulfate, Phosphate, Borate, Nitrate, Hydrogencarbonate und Carbonate.
Weiterhin sind organische Verbindungen geeignet, die mehrwertige Metalle enthalten, wie Acetylacetonate und Alkoholate, wie z.B. Fe(acac)₃, Zr(acac)₄,, Ti(OBu)₄ und Zr(o-Prop)₄.

Der wasserlösliche Vernetzer bewirkt eine Vernetzung der Komponenten A und B, sowohl untereinander als auch zwischeneinander, besonders an der Oberfläche, wodurch, wie in der DE-PS 31 32 976, DE-PS 26 09 144 und US-A 4 959 341 beschrieben ist, die Absorptionseigenschaften verbessert werden.

Als kovalente Vernetzer sind polyfunktionelle Carbonsäuren, Alkohole, Amine, Epoxiverbindungen, Carbonsäureanhydride und Aldehyde, sowie deren Derivate, geeignet. Beispiele sind Citronensäure, Schleimsäure, Weinsäure, Äpfelsäure, Malonsäure, Bernsteinsäure, Glutarsäure, Adipinsäure, Polyethylenglykole, Glycerin, Propandiole, Polyoxypropylenamine, Epichlorhydrin, Ethylenglykoldiglycidylether, Glykoldiglycidylether, Bernsteinsäureanhydrid, Maleinsäureanhydrid, Ethylencarbonat und Propylencarbonat.
Ebenso kommen natürliche Derivate der genannten Verbindungsklassen in Betracht sowie heterofunktionelle Verbindungen mit verschiedenen funktionellen Gruppen der oben genannten Verbindungsklassen.

Der Anteil an Komponente A an dem Verhältnis Komponente A zu Komponente B beträgt 70 bis 99,99 Gew.%, vorzugsweise 75 bis 95 Gew.%. Der Anteil an Komponente B beträgt 0,01 bis 30 Gew.%, vorzugsweise 5 - 25 Gew.%.

Der Zusatz, auch in geringen Mengen, an Komponente B bewirkt eine starke Verbesserung der Absorptionseigenschaften, vor allem des Saugvermögens. Dadurch kann eine überraschend deutliche Verbesserung der Absorptionseigenschaften gegenüber reinem Carboxymethylcellulose-Material (CMC-Material) erzielt werden. Der notwendige Anteil an Komponente B wird durch eine Verarbeitung der Komponenten A und B, beispielsweise in gequollenem Zustand und anschließendes Trocknen noch deutlich reduziert.

Die Menge an Anti-Blocking-Mittel beträgt vorzugsweise zwischen 0,5 und 50 Gew. %, besonders bevorzugt 5 bis 15 Gew. % bezogen auf die Komponenten A und B.

Die Vernetzermenge im Absorber beträgt 0,001 - 10 Gew.%, vorzugsweise 3 - 7 Gew.%, bezogen auf die Komponenten A und B.

Der Zusatz von Matrixmaterial bezogen auf die Komponenten A und B soll zwischen 0,1 - 30 Gew.%, vorzugsweise zwischen 2,5 und 7,5 Gew.-%, betragen.

Das Matrixmaterial verhindert ein Auseinanderfallen des Absorptionsmaterials, wie es bei reinen physikalischen Mischungen wie z. B. US-A-4,952,550 beobachtet wird, und verhindert zusätzlich ein Gelblocking.

Das Zusammenbringen der Polymerkomponenten und das Vermischen der weiteren Komponenten erfolgt bei Temperaturen von 0 °C bis 100 °C, bevorzugt bei Raumtemperatur.

Die vorzugsweise Herstellung des Absorptionsmaterials wird nachfolgend beschrieben.

Stufe 1) Die Komponente A und die Komponente B werden physikalisch in trockener Form bei Raumtemperatur vermischt. Dann läßt man diese unter Rühren in Wasser, bzw. in einer wäßrige Lösung zusammen aufquellen . Nach 15 bis 360 Minuten wird das erhaltene Material bei 40°C bis 180°C im Trockenschrank getrocknet. Das erhaltene Produkt wird anschließend gemahlen.

Stufe 2) Das erhaltene Material wird mit dem Anti-Blocking-Mittel und der Matrix-Komponente vermischt, bis eine homogene Mischung vorliegt. Das Mischen der Komponenten erfolgt in geeigneten Mischern wie Schneckenmischern, Wirbelschichtbettmischern, Scheibenmischern oder Bandmischern.
Die Wärmebehandlung findet bei 25°C bis 180°C, vorzugsweise bei 100°C - 120°C statt. Die Erwärmungsdauer beträgt 5 bis 60 Minuten, vorzugsweise 20 bis 40 Minuten. Zur Wärmebehandlung des Produktes werden gewöhnliche Trockner oder Heizöfen (z.B. Scheibentrockner, Bandtrockner, Wirbelschichtbetttrockner oder Infrarottrockner) eingesetzt.

Stufe 3) Anschließend wird der ionische Vernetzer, vorzugsweise Aluminiumdihydroxyacetat, stabilisiert mit Borsäure, bei Raumtemperatur mit dem erhaltenen Material kräftig gemischt, bis eine homogene Mischung entstanden ist. Zur Fixierung des Vernetzers durch die Matrix wird nochmals auf 25°C bis 180°C, vorzugsweise auf 50°C bis 80°C, für 5 bis 60 Minuten erhitzt, um das Matrixmaterial aufzuschmelzen.

Gegebenenfalls kann, statt der in Stufe 1 beschriebenen Verarbeitung, die Komponente A in eine (z. B. mit Wasser) aufgequollene Komponente B bzw. die Komponente B in die (z. B. mit Wasser) aufgequollenen Komponente A oder die (z. B. mit Wasser) aufgequollene Komponente A in die (z. B. mit Wasser) aufgequollene Komponente B, ggf. unter Zugabe von Wasser oder einer wäßrigen Lösung, eingearbeitet werden.

Nach der Mahlung (Stufe 1) kann das Produkt abgesiebt werden, vorzugsweise auf eine Korngröße von 90 - 630 µm.

Die Einarbeitung der Matrixkomponenten erfolgt vorzugsweise bei Raumtemperatur, die Matrixkomponente kann aber auch als Schmelze eingesetzt werden. Die Mischung in Stufe 2 kann vor der thermischen Modifizierung mit einem vorzugsweise Wasser/Isopropanol-Gemisch versetzt werden, um einen Lösungsvermittler zu haben. Statt dem Wasser/Isopropanol-Gemisch können auch Wasser und andere Gemische von Wasser mit wasserlöslichen organischen Lösungsmitteln eingesetzt werden.
In der EP-PS 0 083 022 wird die Vernetzung eines Absorbers beschrieben, der aus Polyacrylsäure besteht, mit Vernetzern, die wenigstens zwei funktionelle Gruppen enthalten und in der Lage sind, mit den Carboxylgruppen des Polyacrylats zu reagieren. Die Reaktion erfolgt an der Oberfläche der Absorberteilchen. Die DE-PS 33 14 019 und die DE-PS 35 23 617 beschreiben ebenfalls die Oberflächenvernetzung von Polyacrylaten mit Hilfe von Vernetzern, die wenigstens zwei funktionelle Gruppen besitzen. Im Gegensatz zu den erfindungsgemäßen Absorbern werden in diesen Patenten nur Modifizierungen von Polyacrylaten, jedoch nicht von Polysacchariden, in der Hülle beschrieben, wodurch aber keinesfalls Absorber erhalten werden, die ausreichend biologisch abbaubar sind.

Die Einarbeitung des ionischen Vernetzers kann auch direkt in die physikalische Mischung der Stufe 2) erfolgen, worauf dann auf 25°C bis 180°C, vorzugsweise auf 100°C bis 120°C, für 5 bis 120 Minuten, vorzugsweise für 20 bis 60 Minuten erwärmt wird.
Der oben beschriebene Lösungsmittel-Schritt kann bei diesem Verfahren vor oder nach der Vernetzereinarbeitung erfolgen.

Der kovalente Vernetzer kann alternativ und zusätzlich zu dem ionischen Vernetzer zu der Polymermischung vor oder nach der Matrixzugabe zugesetzt werden.
Der kovalente Vernetzer wird in einem vorzugsweise gegebenenfalls Alkohol/Wasser-Gemisch gelöst und der Polymermischung unter schnellem Rühren zugetropft. Die Lösungsmittel-Menge beträgt zwischen 1 und 10 % bezogen auf die Polymermischung. Anschließend wird auf 25°C bis 180°C für 5 bis 120 Minuten erwärmt. Als Lösungsmittel können Wasser und Gemische aus Wasser mit wasserlöslichen organischen Lösungsmitteln verwendet werden.

Gegebenenfalls können die Antiblockingmittel wie auch der kovalente Vernetzer bereits in Stufe 1) zugesetzt werden.

Das erfindungsgemäße Absorptionsmaterial zeigt eine gute biologische Abbaubarkeit gegenüber Produkten, die auf einer Polyacrylsäurebasis beruhen, bei einem, gegenüber bisher bekannten Absorptionsmaterialien auf nativer Basis stark verbesserten Aufnahme- und Saugvermögen für eine 0,9 %ige Kochsalzlösung, auch unter Druck, bei einer überraschend sehr hohen Gelstärke.

| Gelstärke einiger erfindungsgemäßer Absorber sowie einiger marktbekannter Absorber | |
|---|---|
| Produktbezeichnung | Gelstärke (10 Hz) (N/m²) |
| erfindungsgemäße Absorber | |
| Superabsorber aus Bsp. 1 | ≥ 10000 |
| Superabsorber aus Bsp. 3 | ≥ 10000 |
| Superabsorber aus Bsp. 5 | ≥ 10000 |
| Superabsorber aus Bsp. 7 | ≥ 10000 |
| Superabsorber aus Bsp. 9 | ≥ 10000 |

| marktbekannte Absorber | |
|---|---|
| Produkt A | 2450 |
| Produkt B | 4200 |
| Produkt C | 3500 |
| Produkt D | 2700 |
| Produkt E | 4950 |
| Produkt F | 3700 |
| Produkt G | 1575 |
| Produkte A, B, C, D, F und G: vernetzte, teilweise neutralisierte Polyacrylate Produkt E: vernetztes, teilweise neutralisiertes Polyacrylat-Stärke-Pfropfpolymer | |

Weiterhin ist die mechanische Festigkeit (in Bezug auf Entmischung in die Einzelkomponenten) gegenüber den zuvor beschriebenen, auf nachwachsenden Rohstoffen basierenden Absorbern deutlich verbessert.

Die erfindungsgemäße Polymerzusammensetzung kann insbesondere als Absorptionsmaterial als Faser, Film, Pulver oder Granulat eingesetzt werden, um Wasser oder wässrige Flüssigkeiten, wie Urin und Blut, aufzunehmen und ist somit besonders für den Einsatz in Windeln, Tampons, chirurgischen Erzeugnissen, Kabelummantelungen, Kulturgefäßen, Verpackungsmaterialien für Fleisch oder Fisch und absorbierenden Kleidungsstücken geeignet.

Außerdem ist das Material als Speichermedium zur sukzessiven Freisetzung von Wirkstoffen, wie Arzneimitteln, Pestiziden (US 4,818,534; US 4,983,389; US 4,983,390; US 4,985,251) und Riechstoffen geeignet, mit dem Vorteil, daß das Speichermedium abbaubar ist.
Dadurch ergibt sich als weiterer Vorteil, daß der Wirkstoff vollständig freigesetzt wird.

Die wirkstoffhaltigen Depotmaterialien können durch Absorption, vorzugsweise konzentrierter, wässriger oder wasserhaltiger Lösungen in den weitgehend trockenen Absorber und ggf. dessen erneute Trockung hergestellt werden.

Der Wirkstoff kann auch direkt oder als Lösung oder Dispersion bei beliebigen Vorstufen des Herstellungsprozesses der Absorberzusammensetzung zugesetzt werden.

Die wirkstoffhaltigen Depotmaterialien werden in Pulverform oder als Dispersion in hydrophoben Medien, die dispersionsstabilisierende Mittel wie Emulgatoren oder Stabilisatoren enthalten können, oder in Mischung mit anderen Stoffen wie Polysacchariden verwendet.

Beispielsweise wird durch den Zusatz solcher bakterizidhaltiger Depotmaterialien zu Cellulose-, Guar- oder Stärke-produkten oder deren Derivate, wie Carboxymethylcellulose, bei deren Lagerung und Anwendung in wässrigen Medien der Abbau dieser Substanzen über längere Zeit verhindert und wobei durch die Depotwirkung größere Mengen von freiem Wirkstoff in der Lösung vermieden werden können.

### Testmethoden:

### Teebeutel-Test (TBT)

Zur Bestimmung des Absorptionsvermögens wurde ein Teebeutel-Test durchgeführt. Als Prüflösung wurde eine wäßrige 0,9 %ige NaCl-Lösung verwendet.
0,2 g einer Prüfsubstanz (abgesiebt zwischen 90 und 630 µm), die in einem Teebeutel eingewogen wurde, ließ man 10 bzw. 30 Minuten in der Prüflösung aufquellen. Nach fünfminütigem Abtropfen (Maximalwert) wurde in einer Zentrifuge, wie z.B. in einer handelsüblichen Wäscheschleuder, bei 1400 Upm abgeschleudert. Die Flüssigkeitsaufnahme wurde gravimetrisch ermittelt und auf 1 g Substanz umgerechnet. (Retentionswert).

### Absorption under Load (AUL)

Um das Flüssigkeitsaufnahmevermögen unter Druck zu bestimmen, wurde die Absorption under Load , wie in EP-A 0 339 461 beschrieben, bestimmt.
0,16 g Prüfsubstanz (abgesiebt zwischen 300 und 600 µm) ließ man durch Kapillarwirkung für 60 Minuten unter einem Druck von 1,55 kN/m» (99,8 g/in²) in 0,9 %ige NaCl-Lösung quellen. Die Flüssigkeitsaufnahme wurde gravimetrisch ermittelt und auf 1 g Substanz umgerechnet.

### Gelstärke (G')

Um die Gelstärke G' der gequollenen Absorber zu bestimmen, wurde, wie in EP-A 0 339 461 beschrieben, vorgegangen. Gerät: Controlled Stress Rheometer CS 100, Carri-Med Ltd. Dorking/UK.
Meßbedingungen: Plate-Platte-System, Durchmesser 60 mm, Plattenabstand 2 mm, Temperatur 20°C, Drehmoment 1000 - 4000 µNm, Amplitude 1,5 - 5 mrad, Frequenz 10,0 Hz, 28 ml 0,9 % NaCl/g Absorber. Die Angaben erfolgen in N/m².

### Fließtest (FT)

Mittels des Fließtests wurde ermittelt, wie schnell die Produkte die Testflüssigkeit aufnahmen, ob sie Blockphänomene zeigten, vollständig durchgequollen waren und überall benetzt waren. Weiterhin wurde untersucht, ob die Gele fest, klebrig oder locker und separiert vorlagen.

Zur Durchführung des Fließtests wurden ca. 100 mg Substanz auf ein mit Wasser durchtränktes Papiertuch gegeben und verfolgt, wie das Wasser von den Produkten aufgesaugt wurde. Das Absorptionsverhalten wurde nach folgender Notenskala bewertet.
A: zieht schnell an
B: zieht sehr schnell an
C: zieht von Anfang bis Ende durch
D: Gel liegt nach der Wasseraufnahme separiert vor
E : Gelblocking

Die Erfindung wird nachfolgend durch Herstellungs- und Anwendungsbeispiele näher erläutert.

### Vorprodukte

Die nachfolgend angeführten Mischungen werden bei Raumtemperatur (15 bis 20°C) in je 360 ml Wasser eingerührt und drei Stunden stehen gelassen. Danach werden die erhaltenen Gele zwei Stunden bei 100°C im Umlufttrockenschrank ge-trocknet, anschließend gemahlen und auf eine Korngröße von 90 - 630 µm abgesiebt.

### Vorprodukt 1

38 g CMC Walocel 40000 (Natriumcarboxymethylcellulose, Wolff Walsrode), 2 g eines Polyacrylat Superabsorbers (hergestellt nach der DE-OS 40 15 085, Beispiel 4, im folgenden "SAB A" genannt)

### Vorprodukt 2

38 g CMC, (Walocel 30000), 2 g "SAB A"

### Vorprodukt 3

36 g CMC, (Walocel 30000), 4 g "SAB A"

### Vorprodukt 4

32 g CMC, (Walocel 30000), 8 g "SAB A"

### Vorprodukt 5

32 g CMC, (Walocel 30000), 8 g "SAB A", 4 g Cellulosefaser (PWC 500, Fa. Rettenmaier)

### Vorprodukt 6

28,5 g CMC, (Walocel 30000), 9,5 g Guarmehl (Typ 104, Fa. Roeper), 2 g "SAB A"

### Vorprodukt 7 (Vergleichsprodukt)

40 g CMC, (Walocel 40000) ohne Zusatz

### Beispiele

### Beispiel 1

5 g des Vorproduktes 1 werden mit 0,25 g (Cellulose, Durchmesser: 17 µm, Länge: 30 µm) Faser BE 600/30 (Fa. Rettenmaier) und 0,25 g säureterminiertem TONE 230 (ein Umsetzungsprodukt aus TONE 230 Polyol auf der Basis von Caprolacton, Molgewicht 1250 g . mol⁻¹) der Fa. Union Carbide und Maleinsäureanhydrid) kräftig vermischt und anschließend 30 Minuten auf 120°C im Ofen erhitzt. Dann wird mit 0,25 g Al(OH)₂OOCCH₃*1/3 H₃BO₃ versetzt und für eine Stunde auf 50°C im Ofen erhitzt.
TBT (max./ret.) = 48 g/g / 26 g/g; AUL = 18,0 g/g; FT: B C D

### Beispiel 2

5 g des Vorproduktes 2 werden mit 0,25 g Aerosil R 972 (pyrogene Kieselsäure, Teilchendurchmesser: 16 nm Degussa AG), 0,25 g säureterminiertem TONE 230, 0,5 ml Wasser und 1 ml i-Propanol kräftig vermischt und anschließend für 30 Minuten auf 120°C im Ofen erhitzt. Dann wird mit 0,25 g Al(OH)₂OOCCH₃*1/3 H₃BO₃ versetzt und für eine Stunde auf 50°C im Ofen erhitzt.
TBT (max./ret.) = 51 g/g / 28 g/g; AUL = 19,6 g/g; FT: B C D

### Beispiel 3

Es wird wie in Beispiel 2 verfahren, jedoch wird anstelle des Vorproduktes 2 das Vorprodukt 3 eingesetzt; außerdem wird statt des Aerosils R 972 das Aerosil A 200 (pyrogene Kieselsäure, Teilchendurchmesser: 12 nm Degussa AG) verwendet. TBT (max./ret.) = 45 g/g / 27 g/g; AUL = 17,0 g/g; FT: B C D

### Beispiel 4

Es wird wie in Beispiel 2 verfahren, jedoch wird statt des säureterminiertem TONE 230 reines TONE 230 eingesetzt; außerdem wird mit der doppelten Menge Wasser und der doppelten Menge i-Propanol kräftig vermischt.
TBT (max./ret.) = 52 g/g / 29 g/g; AUL = 19,0 g/g; FT: B C D

### Beispiel 5

Es wird wie in Beispiel 2 verfahren, jedoch wird das Vorprodukt 5 eingesetzt.
TBT (max./ret.) = 47 g/g / 27 g/g; AUL = 18,3 g/g; FT: B C D

### Beispiel 6

Es wird wie in Beispiel 3 verfahren, jedoch wird nur die halbe Menge an säureterminiertem TONE 230 eingesetzt, außerdem wird das Aerosil A 200 durch die gleiche Menge der Faser BE 600/30 ersetzt.
TBT (max./ret.) = 52 g/g / 29 g/g; AUL = 18,7 g/g; FT: B C D

### Beispiel 7

Es wird wie in Beispiel 2 verfahren, jedoch werden zusätzlich (vor dem ersten Erhitzen) 0,25 g Faser BE 600/30 eingearbeitet.
TBT (max./ret.) = 49 g/g / 28 g/g; AUL = 18,9 g/g; FT: B C D

### Beispiel 8

Es wird wie in Beispiel 2 verfahren, jedoch wird das Vorprodukt 6 eingesetzt.
TBT (max./ret.) = 38 g/g / 22 g/g; AUL = 16,5 g/g; FT: A C D

### Beispiel 9

Es wird wie in Beispiel 2 verfahren, jedoch wird das Vorprodukt 5 eingesetzt. Außerdem wird die Menge an Aluminiumvernetzer auf 0,2 g reduziert.
TBT (max./ret.) = 49 g/g / 28 g/g; AUL = 16,8 g/g; FT: A C D

### Beispiel 10

100 g des in Beispiel 1 erhaltenen Produktes werden mit 100 ml einer 0,125 %igen wässrigen Lösung von 3,7-Bis (dimethylamino)- phenothiaziniumchlorid gemischt und anschließend für 2 h im Umlufttrockenschrank bei 60°C getrocknet.
200 mg des so erhaltenen Produktes werden in einen Teebeutel gegeben. Dieser wird in ein Becherglas mit 50 ml 0,2 %iger Kochsalzlösung eingehängt. Nach einer Stunde wird der Teebeutel entfernt. Die Färbung der Kochsalzlösung wird beurteilt, dannach wird der Vorgang mit neuer NaCl-Lösung wiederholt.
Auch nach dem 5. Zyklus zeigt die Blaufärbung der Kochsalzlösung die Freisetzung des Wirkstoffs aus der als Speichermedium fungierenden Polymerzusammensetzung an.

### Beispiel 11

Bei der Darstellung von Vorprodukt 1 werden zusätzlich 0,05 g 3,7 - Bis (dimethylamino) - phenothiaziniumchlorid zur Pulvermischung gegeben und wie beschrieben weiter verarbeitet. Aus diesem Vorprodukt wird gemäß Beispiel 1 ein Absorber hergestellt. Der so dargestellte Absorber wird wie in Beispiel 10 beschrieben untersucht. Die erhaltenen Ergebnisse stimmen mit den in Beispiel 10 erhaltenen überein.

### Vergleichsbeispiele

### Vergleichsbeispiel 1

Es wird wie in Beispiel 2 verfahren, jedoch wird das Vorprodukt 7 eingesetzt und die Menge an säureterminiertem TONE 230 halbiert.

### TBT (max./ret.) = 36 g/g / 27 g/g; AUL = 8,0 g/g; FT: E

### Vergleichsbeispiel 2

20 g CMC 30000 werden mit 8 g Isopropanol, 200 g Wasser, 0,4 g Al(OH)₂OOCCH₃*1/3 H₃BO₃ und 0,8 g Essigsäure 4 Stunden bei 50°C gehalten. Anschließend wird bei 80°C getrocknet. TBT (max./ret.) = 16 g/g / 11 g/g; AUL = 8,9 g/g; FT: E

### Vergleichsbeispiele 3, 4

Die Herstellung der Produkte aus den Beispielen 3 und 5 wurden ohne den Zusatz eines Matrixmaterials wiederholt. Die erhaltenen Produkte waren inhomogen, konnten durch Sieben getrennt werden und blockten. Bezüglich des TBT und des AUL-Tests konnten wegen der Inhomogenität der Produkte (Entmischung beim Absieben) keine reproduzierbaren Werte erhalten werden.

### Vergleichsbeispiel 5

60 g CMC 40000 werden mit 1,5 g Ethylencarbonat, 1,5 ml Wasser und 1,5 ml Isopropanol kräftig vermischt; anschließend wird 60 min auf 120°C im Ofen erhitzt. 8 g dieses Produktes werden mit 2 g Favor^{R} 953 (vernetztes, teilneutralisiertes Polyacrylat der Fa. Stockhausen GmbH), 0,5 g TONE 230, 0,5 g Faser BE 600/30 und mit 0,5 g Al(OH)₂OOCCH₃*1/3 H₃BO₃ unter Verwendung von 2 ml Isopropanol und 1 ml Wasser kräftig vermischt und für 60 Min auf 120°C im Ofen erhitzt.
TBT (max./ret.) = 46 g/g / 29 g/g; AUL = 14,4 g/g; FT: B C D

### Vergleichsbeispiel 6

8 g CMC 40000 werden mit 2 g "SAB A", 0,5 g Faser BE 600/30 0,5 g säureterminiertem TONE 230, 0,1 g Aerosil R 972, 2 ml i-Propanol und 1 ml Wasser kräftig vermischt und anschließend 30 Minuten auf 120°C im Ofen erhitzt. Das so erhaltene Produkt wird mit 0,6 g Al(OH)₂OOCCH₃*1/3 H₃BO₃ versetzt und anschließend eine Stunde auf 50°C im Ofen erhitzt.
TBT (max./ret.) = 51 g/g / 36 g/g; AUL = 11,0 g/g; FT: B C D

## Patentansprüche

1. Polymerzusammensetzung, insbesondere Absorptionsmaterialzusammensetzung, im wesentlichen bestehend aus 70 bis 99,99 Gew.-% eines wasserlöslichen und/oder wasserquellbaren Polysaccharids oder Polysaccharidderivats, das gegebenenfalls durch Vernetzung modifiziert worden ist (Komponente A)
und
0,01 bis 30 Gew.-% eines wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(anhydrid), Itaconsäure(anhydrid), Fumarsäure, Vinylsulfonsäure und/oder 2-Acrylamido-2-methylpropansulfonsäure, den Amiden, den N-Alkylderivaten, den N,N'-Dialkylderivaten, den hydroxylgruppenhaltigen Estern und aminogruppenhaltigen Estern dieser polymerisationsfähigen Säuren, wobei 0 bis 98 Gew.-% der Säuregruppen dieser Säuren neutralisiert sind und wobei diese Polymere und /oder Copolymere durch eine mindestens bifunktionelle Verbindung vernetzt sind (Komponente B)
sowie
0,1 bis 30 Gew.-%, bezogen auf die polymeren Komponenten A und B eines organischen Matrixmaterials mit einem Schmelz- bzw. Erweichungspunkt unterhalb von 180° C zur Verhinderung der Entmischung und des Gelblocking
0,001 bis 10 Gew.-%, bezogen auf die beiden polymeren Komponenten A und B, eines ionischen und/oder kovalenten Vernetzers
und
0 bis 50 Gew.-%, bezogen auf die polymeren Komponenten A + B, wenigstens eines Antiblockingmittels auf Basis natürlicher und/oder synthetischer Fasern und/oder Materialien mit großer Oberfläche, erhältlich durch Zusammenbringen der Komponente B mit der Komponenten A in wäßrigem Medium, anschließende Trocknung und Mahlung, Hinzumischung der weiteren Komponenten, Mischung bis zur Homogenität, Durchführung einer Wärmebehandlung und gegebenenfalls nach Zugabe des Vernetzers zwecks dessen Fixierung durch die Matrix eine abschließende Wärmebehandlung.

2. Wirkstoffenthaltende Zusammensetzung, im wesentlichen bestehend aus einer Zusammensetzung nach Anspruch 1 sowie wenigstens einem Wirkstoff, beispielsweise einem Arzneimittel, einem Pestizid, einem Bakterizid und/oder einem Riechstoff, der verzögert freigesetzt wird.

3. Zusammensetzung nach Anspruch 1 oder 2, im wesentlichen bestehend aus 75 - 95 Gew.-% Komponente A, 5 - 25 Gew.-% Komponente B als polymere Komponenten und 2,5 - 7,5 Gew.-%, bezogen auf diese Komponenten, wenigstens eines Matrixmaterials, 3 - 7 Gew.-%, bezogen auf diese Komponenten, wenigstens eines ionischen und/oder kovalenten Vernetzers und 0,5 - 50 Gew.-%, bezogen auf diese Komponenten, wenigstens eines Antiblockingmittels.

4. Zusammensetzung nach Anspruch 3, enthaltend 5 - 15 Gew.-% wenigstens eines Antiblockingmittels.

5. Zusammensetzung nach einem der Ansprüche 1 - 4, dadurch gekennzeichnet, daß die Komponente A ein wasserlösliches und/oder wasserquellbares Polymer auf Basis von Polysacchariden und deren Derivaten, insbesondere Guar-, Stärke- und Zellulosederivaten ist.

6. Zusammensetzung nach Anspruch 5, dadurch gekennzeichnet, daß die Komponente A ein anionisches Derivat von Zellulose, insbesondere von Carboxymethylzellulose ist.

7. Zusammensetzung nach einem der Ansprüche 1 - 6, dadurch gekennzeichnet, daß das Matrixmaterial eine Substanz ist, die bei Raumtemperatur hochviskos ist oder eine wachsartige weiche Konsistenz aufweist.

8. Zusammensetzung nach Anspruch 7, dadurch gekennzeichnet, daß das Matrixmaterial ausgewählt ist aus Triglycerinmonostearat, Rizinusöl und Polycaprolactonen, die gegebenenfalls durch eine Umsetzung mit Maleinsäureanhydrid modifiziert sind.

9. Zusammensetzung nach einem der Ansprüche 1 - 8, dadurch gekennzeichnet, daß die ionischen Vernetzer ausgewählt sind aus Metallverbindungen in Form ihrer Salze mit organischen und anorganischen Säuren.

10. Zusammensetzung nach Anspruch 9, dadurch gekennzeichnet, daß die ionischen Vernetzer ausgewählt sind aus Magnesium-, Calcium-, Aluminium-, Zirkonium-, Eisen-, Titan- und Zinkverbindungen.

11. Zusammensetzung nach einem der Ansprüche 1 - 8, dadurch gekennzeichnet, daß die kovalenten Vernetzer ausgewählt sind aus polyfunktionellen Carbonsäuren, Alkoholen, Aminen, Epoxiverbindungen, Carbonsäureanhydriden und/oder Aldehyden sowie deren Derivaten und heterofunktionellen Verbindungen mit verschiedenen funktionellen Gruppen der genannten Verbindungsklassen.

12. Verfahren zur Herstellung einer Polymerzusammensetzung, insbesondere eines Absorbermaterials, im wesentlichen bestehend aus 70 - 99,99 Gew.-% einer Komponente A, bestehend aus wasserlöslichen und/oder wasserquellbaren Polysacchariden oder Polysaccharidderivaten, die gegebenenfalls durch Vernetzung modifiziert worden sind und 0,01 - 30 Gew.-% einer Komponente B, bestehend aus wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(anhydrid), Itakonsäure(anhydrid), Fumarsäure, Vinylsulfonsäure und/oder 2-Acrylamido-2-methylpropansulfonsäure sowie den Amiden, den N-Alkylderivaten, den N,N'-Dialkylderivaten, hydroxylgruppenhaltigen Estern und aminogruppenhaltigen Estern dieser polymerisationsfähigen Säuren, wobei 0 - 98 % der Säuregruppen dieser Säuren neutralisiert sind, und wobei diese Polymere und/oder Copolymere durch eine mindestens bifunktionelle Verbindung vernetzt sind als polymere Komponenten sowie 0,1- 30 Gew.-%, bezogen auf diese polymeren Komponenten, eines organischen Matrixmaterials mit einem Schmelzpunkt bzw. Erweichungspunkt unterhalb von 180 °C zur Verhinderung der Entmischung und des Gelblockings, 0,001 - 10 Gew.-%, bezogen auf diese beiden polymeren Komponenten, eines ionischen und/oder kovalenten Vernetzers und 0 - 50 Gew.-%, bezogen auf diese polymeren Komponenten, wenigstens eines Antiblockingmittels auf Basis natürlicher und/oder synthetischer Fasern und/oder Materialien mit großer Oberfläche, dadurch gekennzeichnet, daß man diese(s) erhält, indem man die Komponente B mit der Komponente A in wäßrigem Medium zusammenbringt, anschließend trocknet und mahlt, die weiteren Komponenten hinzumischt, bis zur Homogenität mischt und eine Wärmebehandlung durchführt und gegebenenfalls bei Zugabe des Vernetzers nach dieser Wärmebehandlung diesen mit der Matrix durch eine abschließende Wärmebehandlung fixiert.

13. Verfahren nach Anspruch 12, dadurch gekennzeichnet, daß man eine Polymerzusammensetzung, insbesondere ein Absorbermaterial, im wesentlichen bestehend aus 75-95 Gew.-% Komponente A, 5 - 25 Gew.-% Komponente B als polymere Komponenten und 2,5 - 7,5 Gew.-%, bezogen auf diese Komponenten, wenigstens eines Matrixmaterials, 3 - 7 Gew.-%, bezogen auf diese Komponenten, wenigstens eines ionischen und/oder kovalenten Vernetzers und 0,5 - 50 Gew.-%, bezogen auf diese Komponenten, wenigstens eines Antiblockingmittels einsetzt.

14. Verfahren nach Anspruch 13, dadurch gekennzeichnet, daß man 5 - 15 Gew.-% wenigstens eines Antiblockingmittels einsetzt.

15. Verfahren nach Ansprüchen 12 - 14, dadurch gekennzeichnet, daß man als Komponente A Guar-, Stärke- und Zellulosederivate einsetzt.

16. Verfahren nach Anspruch 14, dadurch gekennzeichnet, daß man als Komponente A ein anionisches Derivat von Zellulose, insbesondere von Carboxymethylzellulose einsetzt.

17. Verfahren nach einem der Ansprüche 12 - 16, dadurch gekennzeichnet, daß man als Matrixmaterial eine Substanz einsetzt, die bei Raumtemperatur hochviskos ist oder eine wachsartige weiche Konsistenz aufweist.

18. Verfahren nach Anspruch 17, dadurch gekennzeichnet, daß das Matrixmaterial ausgewählt ist aus Triglycerinmonostearat, Rizinusöl und Polycaprolactonen, die gegebenenfalls durch eine Umsetzung mit Maleinsäureanhydrid modifiziert sind.

19. Verfahren nach einem der Ansprüche 12 - 18, dadurch gekennzeichnet, daß die ionischen Vernetzer ausgewählt sind aus Metallverbindungen in Form ihrer Salze mit organischen und anorganischen Säuren.

20. Verfahren nach Anspruch 19, dadurch gekennzeichnet, daß die ionischen Vernetzer ausgewählt sind aus Magnesium-, Calcium-, Aluminium-, Zirkonium-, Eisen-, Titan- und Zinkverbindungen.

21. Verfahren nach einem der Ansprüche 12 - 18, dadurch gekennzeichnet, daß die kovalenten Vernetzer ausgewählt sind aus polyfunktionellen Carbonsäuren, Alkoholen, Aminen, Epoxiverbindungen, Carbonsäureanhydriden und/oder Aldehyden sowie deren Derivaten und heterofunktionellen Verbindungen mit verschiedenen funktionellen Gruppen der genannten Verbindungsklassen.

22. Verfahren nach Ansprüchen 12 - 21, dadurch gekennzeichnet, daß man die Komponente B in der Weise mit Komponente A kontaktiert, indem man beide Komponenten vermischt, diese in wäßrigem Medium zusammenbringt, dann in einer wasserenthaltenden Lösung oder Dispersion aufquillt, trocknet und mahlt.

23. Verfahren nach Anspruch 22, dadurch gekennzeichnet, daß man entweder beide polymeren Komponenten in trockener Form mischt oder eine trockene polymere Komponente mit der anderen polymeren Komponenten in bereits einer durch eine wasserenthaltende Lösung oder Dispersion aufgequollenen Form vermischt.

24. Verfahren nach einem der Ansprüche 12 - 22, dadurch gekennzeichnet, daß man die Komponente B in der Weise mit der Komponenten A kontaktiert, indem man die durch eine wasserenthaltende Lösung oder Dispersion aufgequollene Komponente B mit einer durch eine wasserenthaltende Lösung oder Dispersion aufgequollene Komponente A gegebenenfalls unter Zugabe einer wasserenthaltenden Lösung oder Dispersion mischt, die Komponenten trocknet und mahlt.

25. Verfahren nach einem der Ansprüche 12 - 24, dadurch gekennzeichnet, daß das Zusammenbringen der Polymerkomponenten und das Vermischen der weiteren Komponenten bei Temperaturen von 0 °C bis 100 °C erfolgt.

26. Verfahren nach Anspruch 25, dadurch gekennzeichnet, daß das Zusammenbringen der Polymerkomponenten bei Raumtemperatur erfolgt.

27. Verfahren nach einem der Ansprüche 12 - 26, dadurch gekennzeichnet, daß die Trocknung bei 40 °C bis 180 °C durchgeführt wird und das gemahlene Produkt auf eine Korngröße von 90 bis 630 µm abgesiebt wird.

28. Verfahren nach einem der Ansprüche 12 - 27, dadurch gekennzeichnet, daß man die Wärmebehandlung bei 25 °C bis 180 °C und die abschließende Wärmebehandlung bei 25 °C bis 180 °C durchführt.

29. Verfahren nach Anspruch 28, dadurch gekennzeichnet, daß man die Wärmebehandlung bei 100 - 120 °C und die abschließende Wärmebehandlung bei 50 - 80 °C durchführt.

30. Verfahren nach einem der Ansprüche 12 - 29, dadurch gekennzeichnet, daß man im Anschluß an das Vermahlen die weiteren Komponenten in Gegenwart einer wasserenthaltenden Lösung oder Dispersion und einem wasserenthaltenden organischen Lösemittel zumischt.

31. Verfahren nach Anspruch 30, dadurch gekennzeichnet, daß man die weiteren Komponenten in Gegenwart von Wasser oder einem Gemisch aus Wasser und einem wasserenthaltenden organischen Lösemittel zumischt.

32. Verfahren nach einem der Ansprüche 12 - 31, dadurch gekennzeichnet, daß man den Vernetzer in einem Gemisch aus Wasser und/oder einem wasserhaltigen organischen Lösemittel löst oder dispergiert und den zu kontaktierenden Polymerkomponenten oder den weiteren Komponenten vor der Wärmebehandlung zuführt.

33. Verfahren zur Herstellung einer Depotmaterialzusammensetzung nach Anspruch 2, dadurch gekennzeichnet, daß der Wirkstoff
- entweder aus wäßrigen oder wasserenthaltenden Lösungen von der Zusammensetzung nach Ansprüchen 1 und 3 bis 11 absorbiert wird und gegebenenfalls erneut getrocknet wird
- oder als Lösung oder Dispersion bei beliebigen Vorstufen des Herstellungsverfahrens der besagten Zusammensetzung zugesetzt wird.

34. Verwendung der Zusammensetzung nach den Ansprüchen 1 und 3 bis 11 oder hergestellt nach Ansprüchen 12 bis 32 als Faser, Film, Pulver oder Granulat zur Absorption von wasserenthaltenden Lösungen oder Dispersionen und (Körper-)Flüssigkeiten in chemisch-technischen Produkten, in Kulturgefäßen, zur Bodenverbesserung, als Kabelummantelung oder in Hygieneartikeln.

35. Verwendung der Zusammensetzung nach Anspruch 34 in Verpackungsmaterialien, Tampons, Windeln oder Tierhygieneartikeln.

36. Verwendung der Zusammensetzung nach Anspruch 2 oder hergestellt nach Anspruch 33 in Pulverform oder als Dispersion in hydrophoben Medien gegebenenfalls in Kombination mit Dispersionsstabilisatoren oder in Mischung mit anderen Stoffen.

37. (Tier-)Hygieneartikel enthaltend eine Zusammensetzung nach Ansprüchen 1 bis 11 oder hergestellt gemäß Ansprüchen 12 bis 32.

38. Chemisch-technische Produkte enthaltend eine Zusammensetzung nach Ansprüchen 1 bis 11 oder hergestellt gemäß Ansprüchen 12 bis 32.

## Claims

1. A polymer composition, in particular absorbent composition, substantially consisting of 70 to 99.99%-wt. of a water-soluble and/or water-swellable polysaccharide or polysaccharide derivative which has optionally been modified by cross-linkage (component A)
and
0.01 to 30%-wt. of a water-swellable, synthetic polymer and/or copolymer of (meth-)acrylic acid, (meth-)acrylonitrile, (meth)acrylamide, vinyl acetate, vinyl pyrrolidone, vinyl pyridine, maleic acid (anhydride), itaconic acid (anhydride), fumaric acid, vinyl sulfonic acid, and/or 2-acrylamido-2-methylpropane sulfonic acid, the amides, the N-alkyl derivatives, the N,N'-dialkyl derivatives, the hydoxyl group-containing esters and amino group-containing esters of these polymerizable acids, with 0 to 98%-wt. of the acid groups of these acids being neutralized, and these polymers and/or copolymers being cross-linked by an at least bifunctional compound (component B)
and
0.1 to 30%-wt., relative to the polymer components A and B, of an organic matrix material having a melting or softening point of below 180°C for the prevention of separation and gel blocking,
0.001 to 10%-wt., relative to the two polymer components A and B, of an ionic and/or covalent cross-linking agent,
and
0 to 50%-wt., relative to the polymer components A + B, of at least one anti-blocking agent based on natural and/or synthetic fibers and/or large-surface materials, obtainable by bringing component B together with component A in aqueous medium, subsequent drying and grinding, adding the other components, mixing up to homogeneity, carrying out a heat treatment, and, after addition of the cross-linking agent, optionally carrying out a final heat treatment for its fixation by the matrix.

2. An active substance-containing composition substantially consisting of a composition according to claim 1 and at least one active substance, for example, a drug, a pesticide, a bactericide, and/or a perfume, which is released in a retarded manner.

3. The composition according to claim 1 or 2 substantially consisting of 75 - 95%-wt. of component A, 5 - 25%-wt. of component B, as polymer components, and 2.5 - 7.5%-wt., relative to these components, of at least one matrix material, 3 - 7%-wt., relative to these components, of at least one ionic and/or covalent cross-linking agent, and 0.5 - 50%-wt., relative to these components, of at least one anti-blocking agent.

4. The composition according to claim 3 comprising 5 - 15%-wt. of at least one anti-blocking agent.

5. The composition according to any one of claims 1 - 4 characterized in that component A is a water-soluble and/or water-swellable polymer based on polysaccharides and their derivatives, in particular guar, starch, and cellulose derivatives.

6. The composition according to claim 5 characterized in that component A is an anionic derivative of cellulose, in particular of carboxymethylcellulose.

7. The composition according to any one of claims 1 - 6 characterized in that the matrix material is a substance which is highly viscous or has a wax-like soft consistency at room temperature.

8. The composition according to claim 7 characterized in that the matrix material is selected from triglycerol monostearate, castor oil and polycaprolactones, which are optionally modified by a reaction with maleic anhydride.

9. The composition according to any one of claims 1 - 8 characterized in that the ionic cross-linking agents are selected from metallic compounds in the form of their salts with organic and inorganic acids.

10. The composition according to claim 9 characterized in that ionic cross-linking agents are selected from magnesium, calcium, aluminum, zirconium, iron, titanium, and zinc compounds.

11. The composition according to any one of claims 1 - 8 characterized in that the covalent cross-linking agents are selected from polyfunctional carboxylic acids, alcohols, amines, epoxy compounds, carboxylic acid anhydrides, and/or aldehydes as well as their derivatives and heterofunctional compounds with different functional groups of the mentioned compound classes.

12. A process for the production of a polymer composition, in particular of an absorbent, substantially consisting of 70-99.99%-wt. of a component A consisting of water-soluble and/or water-swellable polysaccharides or polysaccharide derivatives which have optionally been modified by cross-linkage; and 0.01-30%-wt. of a component B consisting of water-swellable, synthetic polymers and/or copolymers of (meth-)acrylic acid, (meth-) acrylonitrile, (meth-)acrylamide, vinyl acetate, vinyl pyrrolidone, vinyl pyridine, maleic acid (anhydride), itaconic acid (anhydride), fumaric acid, vinyl sulfonic acid, and/or 2-acrylamido-2-methylpropane sulfonic acid, as well as the amides, the N-alkyl derivatives, the N,N'-dialkyl derivatives, hydroxyl group-containing esters and amino group-containing esters of these polymerizable acids, with 0 - 98% of the acid groups of these acids being neutralized, and these polymers and/or copolymers being cross-linked by an at least bifunctional compound, used as polymer components; and 0.1 - 30%-wt., relative to these polymer components, of an organic matrix material having a melting or softening point of below 180°C to prevent separation and gel blocking; 0.001 - 10%-wt., relative to these two polymer components, of an ionic and/or covalent cross-linking agent; and 0 - 50%-wt., relative to these polymer components, of at least one anti-blocking agent based on natural and/or synthetic fibers and/or large-surface materials, which process is characterized in that said polymer composition/ absorbent is obtained by bringing component B together with component A in aqueous medium, subsequent drying and grinding, admixing the further components, mixing up to homogeneity, and carrying out a heat treatment, and, after said heat treatment, during addition of the cross-linking agent, optionally carrying out a final heat treatment to fix it by the matrix.

13. The process according to claim 12 characterized in that a polymer composition, in particular an absorbent, is used which substantially consists of 75 - 95%-wt. of component A, 5 - 25%-wt. of component B, used as polymer components, and 2.5-7.5%-wt., relative to these components, of at least one matrix material, 3 - 7%-wt., relative to these components, of at least one ionic and/or covalent cross-linking agent, and 0.5 - 50%-wt., relative to these components, of at least one anti-blocking agent.

14. The process according to claim 13 characterized in that 5-15%-wt. of at least one anti-blocking agent is used.

15. The process according to claims 12 - 14 characterized in that guar, starch, and cellulose derivatives are used component A.

16. The process according to claim 14 characterized in that an anionic derivative of cellulose, in particular of carboxymethylcellulose, is used as component A.

17. The process according to any one of claims 12 - 16 characterized in that a substance which is highly viscous or has a wax-like soft consistency at room temperature is used as matrix material.

18. The process according to claim 17 characterized in that the matrix material is selected from triglycerol monostearate, castor oil and polycaprolactones, which are optionally modified by a reaction with maleic anhydride.

19. The process according to any one of claims 12 - 18 characterized in that the ionic cross-linking agents are selected from metallic compounds in the form of their salts with organic and inorganic acids.

20. The process according to claim 19 characterized in that the ionic cross-linking agents are selected from magnesium, calcium, aluminum, zirconium, iron, titanium, and zinc compounds.

21. The process according to any one of claims 12 - 18 characterized in that the covalent cross-linking agents are selected from polyfunctional carboxylic acids, alcohols, amines, epoxy compounds, carboxylic acid anhydrides, and/or aldehydes as well as their derivatives and heterofunctional compounds with different functional groups of the mentioned compound classes.

22. The process according to claims 12 - 21 characterized in that component B is contacted with component A in such a way that the two components are mixed, brought together in aqueous medium, then swollen in a hydrous solution or dispersion, dried and ground.

23. The process according to claim 22 characterized in that either both polymer components are mixed in dry form or that one dry polymer component is mixed with the other polymer component which is in an already swollen form by means of a hydrous solution or dispersion.

24. The process according to any one of claims 12 - 22 characterized in that component B is contacted with component A in such a way that component B which is swollen by a hydrous solution or dispersion is mixed with a component A which is swollen by a hydrous solution or dispersion, optionally under the addition of a hydrous solution or dispersion, and that the components are dried and ground.

25. The process according to any one of claims 12 - 24 characterized in that the contact of the polymer components and the mixing of the other components is effected at temperatures of 0°C to 100°C.

26. The process according to claim 25 characterized in that the contact of the polymer components is effected at room temperature.

27. The process according to any one of claims 12 - 26 characterized in that drying is effected at 40°C to 180°C and the ground product is screened to a particle size of 90 to 630 µm.

28. The process according to any one of claims 12 - 27 characterized in that the heat treatment is carried out at 25°C to 180°C and the final heat treatment is carried out at 25°C to 180°C.

29. The process according to claim 28 characterized in that the heat treatment is carried out at 100°C - 120°C and the final heat treatment is carried out at 50°C - 80°C.

30. The process according to any one of claims 12 - 29 characterized in that, following the grinding, the other components are admixed in the presence of a hydrous solution or dispersion and a hydrous organic solvent.

31. The process according to claim 30 characterized in that the further components are admixed in the presence of water or a mixture of water and a hydrous organic solvent.

32. The process according to any one of claims 12 - 31 characterized in that the cross-linking agent is dissolved or dispersed in a mixture of water and/or a hydrous organic solvent and added to the polymer components to be contacted or to the other components, prior to the heat treatment.

33. A process for the production of a depot material composition according to claim 2 characterized in that the active substance
- is either absorbed by the composition according to claims 1 and 3 to 11 from aqueous or hydrous solutions and optionally dried again,
- or added to said composition as a solution or dispersion in any of the preceding stages of the production process.

34. The use of the composition according to claims 1 and 3 to 11 or manufactured according to claims 12 to 32 as fiber, film, powder, or granular material for the absorption of hydrous solutions or dispersions, and (body) fluids in chemico-technical products, in culture pots, for soil conditioning, as cable sheathing, or in hygiene products.

35. The use of the composition according to claim 34 in packaging materials, tampons, diapers, or in products for animal hygiene.

36. The use of the composition according to claim 2 or manufactured according to claim 33 in the form of a powder or as a dispersion in hydrophobic media, optionally in combination with dispersion stabilizers or in admixture with other substances.

37. (Animal) Hygiene items comprising a composition according to claims 1 to 11 or manufactured according to claims 12 to 32.

38. Chemico-technical products comprising a composition according to claims 1 to 11 or manufactured according to claims 12 to 32.

## Revendications

1. Composition de polymère, plus particulièrement, composition de matériau d'absorption, qui se compose essentiellement de
70 à 99,99% en poids d'un polysaccharide ou d'un dérivé d'un polysaccharide, soluble dans l'eau et/ou gonflable à l'eau, qui a éventuellement été modifié par réticulation (composant A)
et
0,01 à 30% en poids d'un polymère et/ou copolymère synthétique, gonflable à l'eau, de l'acide (méth)acrylique, du (méth)acrylonitrile, du (méth)acrylamide, de l'acétate de vinyle, de la vinylpyrrolidone, de la vinylpyridine, de l'acide (anhydride) maléique, de l'acide (anhydride) itaconique, de l'acide fumarique, de l'acide vinylsulfonique et/ou de l'acide 2-acrylamido-2-méthylpropanesulfonique, des amides, des dérivés N-alkyliques, des dérivés N,N'-dialkyliques, des esters contenant des radicaux hydroxyle et des esters contenant des radicaux amino de ces acides polymérisables, ou de 0 à 98% en poids des radicaux acide de ces acides sont neutralisés et où ces polymères et/ou copolymères sont réticulés par au moins un composé bifonctionnel (composant B),
ainsi que
0,1 à 30% en poids, par rapport aux composants polymériques A et B, d'une matière formant matrice organique d'un point de fusion ou d'un point de ramollissement inférieur à 180°C, en vue d'empêcher la démixtion et le blocage du gel,
0,001 à 10% en poids, par rapport aux deux composants polymériques A et B, d'un agent de réticulation ionique et/ou covalent
et
0 à 50% en poids, par rapport aux composants polymériques A + B, d'au moins un agent antiblocage à base de fibres naturelles et/ou synthétiques et/ou de matériaux à grande surface, que l'on peut obtenir par la réunion du composant B et du composant A, dans un milieu aqueux, le séchage et le broyage subséquents, l'introduction sous mélange des autres composants, mélange jusqu'à homogénéité, entreprise d'un traitement thermique et éventuellement, après l'addition de l'agent de réticulation, en vue de sa fixation dans la matrice, un traitement thermique subséquent.

2. Composition contenant des principes actifs, essentiellement constituée d'une composition suivant la revendication 1, comme aussi d'au moins un principe actif, par exemple, un médicament, un pesticide, un bactéricide et/ou un parfum, qui est libéré de manière retardée.

3. Composition suivant la revendication 1 ou 2, essentiellement constituée de 75 à 95% en poids de composant A, de 5 à 25% en poids de composant B, à titre de composants polymériques et de 2,5 à 7,5% en poids, par rapport à ces composants, d'au moins une matière formant matrice, de 3 à 7% en poids, par rapport à ces composants, d'au moins un agent de réticulation ionique et/ou covalent et de 0,5 à 50% en poids, par rapport à ces composants, d'au moins un agent antiblocage.

4. Composition suivant la revendication 3, qui contient de 5 à 15% en poids d'au moins un agent antiblocage.

5. Composition suivant l'une quelconque des revendications 1 à 4, caractérisée en ce que le composant A est un polymère soluble dans l'eau et/ou gonflable à l'eau, à base de polysaccharides et de leurs dérivés, plus particulièrement, des dérivés de la gomme Guar, d'amidon et de la cellulose.

6. Composition suivant la revendication 5, caractérisée en ce que le composant A est un dérivé anionique de la cellulose, plus particulièrement, de la carboxyméthylcellulose.

7. Composition suivant l'une quelconque des revendications 1 à 6, caractérisée en ce que la matière formant matrice est une substance extrêmement visqueuse ou qui présente une consistance molle, analogue à celle d'une cire, à la température ambiante.

8. Composition suivant la revendication 7, caractérisée en ce que la matière formant matrice est choisie parmi le monostéarate de triglycérine, l'huile de ricin et les polycaprolactones, qui ont éventuellement été modifiés par réaction avec de l'anhydride maléique.

9. Composition suivant l'une quelconque des revendications 1 à 8, caractérisée en ce que l'agent de réticulation ionique est choisi parmi des composés de métaux, sous la forme de leurs sels avec des acides organiques et inorganiques.

10. Composition suivant la composition 9, caractérisée en ce que l'agent de réticulation ionique est choisi parmi les composés du magnésium, du calcium, de l'aluminium, du zirconium, du fer, du titane et du zinc.

11. Composition suivant l'une quelconque des revendications 1 à 8, caractérisée en ce que l'agent de réticulation covalent est choisi parmi des acides carboxyliques polyfonctionnels, des alcools, des amines, des composés du type époxy, des anhydrides d'acides carboxyliques et/ou des aldéhydes, comme aussi leurs dérivés et des composés hétérofonctionnels avec divers radicaux fonctionnels des classes des composés susmentionnées.

12. Procédé de préparation d'une composition de polymère, plus particulièrement, composition de matériau d'absorption, qui se compose essentiellement de 70 à 99,99% en poids d'un polysaccharide ou d'un dérivé d'un polysaccharide, soluble dans l'eau et/ou gonflable à l'eau, qui a éventuellement été modifié par réticulation (composant A) et 0,01 à 30% en poids d'un polymère et/ou copolymère synthétique, gonflable à l'eau, de l'acide (méth)acrylique, du (méth)acrylonitrile, du (méth)acrylamide, de l'acétate de vinyle, de la vinylpyrrolidone, de la vinylpyridine, de l'acide (anhydride) maléique, de l'acide (anhydride) itaconique, de l'acide fumarique, de l'acide vinylsulfonique et/ou de l'acide 2-acrylamido-2-méthylpropanesulfonique, des amides, des dérivés N-alkyliques, des dérivés N,N'-dialkyliques, des esters contenant des radicaux hydroxyle et des esters contenant des radicaux amino de ces acides polymérisables, ou de 0 à 98% en poids des radicaux acide de ces acides sont neutralisés et où ces polymères et/ou copolymères sont réticulés par au moins un composé bifonctionnel (composant B), ainsi que 0,1 à 30% en poids, par rapport aux composants polymériques A et B, d'une matière formant matrice organique d'un point de fusion ou d'un point de ramollissement inférieur à 180°C, en vue d'empêcher la démixtion et le blocage du gel, 0,001 à 10% en poids, par rapport aux deux composants polymériques A et B, d'un agent de réticulation ionique et/ou covalent et 0 à 50% en poids, par rapport aux composants polymériques A + B, d'au moins un agent antiblocage à base de fibres naturelles et/ou synthétiques et/ou de matériaux à grande surface, caractérisé en ce que l'on obtient, pour autant que l'on réunisse le composant B au composant A dans un milieu aqueux, on le sèche et on le broie ensuite, on y ajoute sous mélange les autres composants, on poursuit le mélange jusqu'à l'homogénéité et on entreprend un traitement thermique et éventuellement, lors de l'addition de l'agent de réticulation après ce traitement thermique, on fixe celui-ci à la matrice par un traitement thermique subséquent.

13. Procédé suivant la revendication 12, caractérisé en ce que l'on utilise une composition de polymère, plus particulièrement, un matériau absorbant, que se compose essentiellement de 75 à 95% en poids de composant A, de 5 à 25% en poids de composant B, à titre de composants polymériques et de 2,5 à 7,5% en poids, par rapport à ces composants, d'au moins une matière formant matrice, de 3 à 7% en poids, par rapport à ces composants, d'au moins un agent de réticulation ionique et/ou covalent et de 0,5 à 50% en poids, par rapport à ces composants, d'au moins un agent antiblocage.

14. Composition suivant la revendication 13, caractérisée en ce que l'on utilise de 5 à 15% en poids d'au moins un agent antiblocage.

15. Procédé suivant l'une quelconque des revendications 12 à 14, caractérisé en ce que l'on utilise des dérivés de gomme Guar, d'amidon et de cellulose, à titre de composant A.

16. Procédé suivant la revendication 14, caractérisé en ce que l'on utilise, à titre de composant A, un dérivé anionique de la cellulose, plus particulièrement, de la carboxyméthylcellulose.

17. Procédé suivant l'une quelconque des revendications 12 à 16, caractérisé en ce que l'on utilise, à titre de matière formant matrice, est une substance extrêmement visqueuse ou qui présente une consistance molle, analogue à celle d'une cire, à la température ambiante.

18. Procédé suivant la revendication 17, caractérisé en ce que la matière formant matrice est choisie parmi le monostéarate de triglycérine, l'huile de ricin et les polycaprolactones, qui ont éventuellement été modifiés par réaction avec de l'anhydride maléique.

19. Procédé suivant l'une quelconque des revendications 12 à 18, caractérisé en ce que l'agent de réticulation ionique est choisi parmi des composés de métaux, sous la forme de leurs sels avec des acides organiques et inorganiques.

20. Procédé suivant la revendication 19, caractérisé en ce que l'agent de réticulation ionique est choisi parmi les composés du magnésium, du calcium, de l'aluminium, du zirconium, du fer, du titane et du zinc.

21. Procédé suivant l'une quelconque des revendications 12 à 18, caractérisé en ce que l'agent de réticulation covalent est choisi parmi des acides carboxyliques polyfonctionnels, des alcools, des amines, des composés du type époxy, des anhydrides d'acides carboxyliques et/ou des aldéhydes, comme aussi leurs dérivés et des composés hétérofonctionnels avec divers radicaux fonctionnels des classes des composés susmentionnées.

22. Procédé suivant l'une quelconque des revendications 12 à 21, caractérisé en ce que l'on met le composant B en contact avec le composant A, pour autant que l'on mélange les deux composants, on réunisse ceux-ci dans un milieu aqueux, puis on les gonfle dans une dispersion ou une solution contenant de l'eau, on les sèche et on les broie.

23. Procédé suivant la revendication 22, caractérisé en ce que l'on mélange les deux composants polymériques sous forme sèche, ou bien on mélange un composant polymérique sec à l'autre composant polymérique qui se trouve déjà sous forme gonflée, par une dispersion ou une solution contenant de l'eau.

24. Procédé suivant l'une quelconque des revendications 12 à 22, caractérisé en ce que l'on met le composant B en contact avec le composant A, pour autant que l'on mélange le composant B gonflé par la dispersion ou la solution contenant de l'eau au composant A gonflé par la dispersion ou la solution contenant de l'eau, éventuellement sous addition d'une dispersion ou d'une solution contenant de l'eau, on sèche les composants et on les broie.

25. Procédé suivant l'une quelconque des revendications 12 à 24, caractérisé en ce que la réunion des composants polymériques et le mélanges des autres composants s'effectuent à des températures de 0°C à 100°C.

26. Procédé suivant la revendication 25, caractérisé en ce que l'on entreprend la réunion des composants polymériques à la température ambiante.

27. Procédé suivant l'une quelconque des revendications 12 à 26, caractérisé en ce que l'on opère le séchage à 40°C à 180°C et on crible le produit broyé jusqu'à une granulométrie de 90 à 630 µm.

28. Procédé suivant l'une quelconque des revendications 12 à 27, caractérisé en ce que l'on entreprend le traitement thermique à 25 à 180°C et le traitement thermique subséquent à 25°C à 180°C.

29. Procédé suivant la revendication 28, caractérisé en ce que l'on entreprend le traitement thermique à 100-120°C et le traitement thermique subséquent à 50-80°C.

30. Procédé suivant l'une quelconque des revendications 12 à 29, caractérisé en ce que, subséquemment au broyage, on ajoute sous mélange les autres composants en présence d'une dispersion ou d'une solution contenant de l'eau.

31. Procédé suivant la revendication 30, caractérisé en ce que l'on ajouté sous mélange les autres composants en présence d'eau ou d'un mélange d'eau et d'un solvant organique contenant de l'eau.

32. Procédé suivant l'une quelconque des revendications 12 à 31, caractérisé en ce que l'on dissout ou disperse l'agent de réticulation dans un mélange d'eau et/ou d'un solvant organique contenant de l'eau et on ajoute les composants polymériques à mettre en contact ou les autres composants avant le traitement thermique.

33. Procédé de préparation d'une composition de matériau dépôt suivant la revendication 2, caractérisé en ce que le principe actif
- est absorbé à partir de solutions aqueuses ou contenant de l'eau de la composition suivant les revendications 1 et 3 à 11 et on opère éventuellement un nouveau séchage
- ou bien on l'ajoute sous forme de solution ou de dispersion à n'importe quelles étapes préalables du procédé de préparation de la composition concernée.

34. Utilisation de la composition suivant l'une quelconque des revendications 1 et 3 à 11 ou préparée suivant les revendications 12 à 32, sous forme de fibres, de films, de poudres, ou de granulés, pour l'absorption de dispersions ou de solutions contenant de l'eau et des liquides (corporels) dans des produits chimicotechniques, dans des récipients de culture, pour l'amendement du sol ou de la terre, à titre de revêtements ou de gaines de câbles, ou dans des articles hygiéniques.

35. Utilisation de la composition suivant la revendication 34 dans des matériaux d'emballage, des tampons, des couches ou des articles pour l'hygiène animale.

36. Utilisation de la composition suivant la revendication 2 ou préparée suivant la revendication 33, sous la forme de poudre ou sous la forme de dispersion dans des milieux hydrophobes, éventuellement en combinaison avec des stabilisateurs de dispersion ou en mélange à d'autres matières.

37. Articles pour l'hygiène (animale) contenant une composition suivant les revendications 1 à 11, ou préparée suivant les revendications 12 à 32.

38. Produits chimicotechniques contenant une composition suivant l'une quelconque des revendications 1 à 11, ou préparée suivant les revendications 12 à 32.
